## Europäisches Patentamt
## European Patent Office
## Office européen des brevets

(19)

(11) Numéro de publication: **0 267 077**
**A1**

(12)
# DEMANDE DE BREVET EUROPEEN

(21) Numéro de dépôt: 87402213.0

(22) Date de dépôt: 06.10.87

(51) Int. Cl.4: **A 61 N 1/44**

(30) Priorité: 06.10.86 FR 8614074

(43) Date de publication de la demande:
11.05.88 Bulletin 88/19

(84) Etats contractants désignés:
AT BE CH DE ES GB GR IT LI LU NL SE

(71) Demandeur: **Breton, Jacques Léon Georges**
**9, Avenue de Gradignan**
**F-33600 Pessac Gironde (FR)**

(72) Inventeur: **Breton, Jacques Léon Georges**
**9, Avenue de Gradignan**
**F-33600 Pessac Gironde (FR)**

(74) Mandataire: **Cabinet Pierre HERRBURGER**
**115, Boulevard Haussmann**
**F-75008 Paris (FR)**

(54) **Cabine pour aéro-ionisation intensive, à transfert et capture contrôlés d'ions oxygène négatifs.**

(57) L'invention concerne les dispositifs de type cabine d'aéro-ionisation négative, permettant d'assurer à un patient placé dans la cabine la capture et l'écoulement des charges électriques injectées par un générateur extérieur dans l'atmosphère de cette cabine, en vue d'obtenir les effets hygiéniques ou thérapeutiques résultants .

Ce dispositif comporte un générateur d'ions oxygène négatifs (8) décrit dans la demande de Brevet français No86 12448 , alimenté en air réchauffé, ainsi qu'une entrée supplémentaire d'oxygène (11), les ions produits (13) étant injectés dans la cabine (18) .

Le patient dévêtu (21), placé au centre de la cabine à parois isolantes (externe conductrice (22) à la masse, interne isolante (23) chargée négativement), collecte et capte les ions présents qu'il écoule vers la masse (17) par l'entremise de la plaque plantaire (7) conductrice .

Ces caractéristiques autorisent la production et l'observation d'effets hygiéniques ou thérapeutiques sur les patients justiciables, effectuant un nombre suffisant de séjours dans la cabine .

Figure 1

EP 0 267 077 A1

# Description

"CABINE POUR AERO-IONISATION INTENSIVE, A TRANSFERT ET CAPTURE CONTROLES D'IONS OXYGENE NEGATIFS".

La présente invention concerne les dispositifs du type "cabine d'aéro-ionisation",permettant d'obtenir à l'intérieur d'une enceinte close de faible volume -aisée à transporter et installer-une très importante concentration d'ions oxygène négatifs,destinés au patient placé à l'intérieur de l'enceinte en vue d'une action thérapeutique ou hygiénique .

Les dispositifs connus de ce genre sont généralement constitués d'une enceinte de grand volume,dont la face interne des parois est conductrice,et dans laquelle le patient est placé sans relation électrique définie avec le sol (potentiel de référence)

Tous ces dispositifs présentent des défauts ou inconvénients qui en limitent ou en annulent l'intérêt et les performances dans leur domaine d'application :

- le maintien de la haute densité ionique nécessaire exige en effet que la face interne de l'enceinte soit d'un haut isolement électrique: même une faible conductivité suffit alors à assurer la capture intense des ions produits et leur fuite au sol, avec l'effondrement corrélatif de la densité de charges au centre de l'enceinte ;

- à l'inverse,le patient placé au centre de l'enceinte et isolé par son support (siège,couchette,etc...) acquiert dès son installation une charge négative constante et statique s'opposant à toute capture ultérieure d'ions par répulsion superficielle :cet effet s'oppose alors définitivement -aussi longtemps que dure le séjour du patient dans l'enceinte- au résultat thérapeutique ou hygiénique recherché .Une telle situation est d'ailleurs entièrement confirmée par l'expérience,et conforme aux lois physiques régissant les mécanismes intervenant .

Le dispositif suivant l'invention permet d'éviter totalement ces défauts,et d'assurer précisément le respect nécessaire des règles théoriques en vue du résultat recherché .

Ce dispositif comporte en effet une armature métallique extérieure reliée au sol,à laquelle est fixée par des sangles conductrices une "cabine" en tissu plastifié dont la face interne est parfaitement isolante, tandis que la face externe a été rendue conductrice par métallisation de surface et est connectée au sol. Le "sol" de la cabine est également constitué du même tissu.Au centre de cette surface au sol est disposée une mince feuille de caoutchouc graphité conducteur,dont la surface est de l'ordre de la surface plantaire des pieds du patient;cette feuille conductrice est reliée au sol et permet ainsi le retour des charges collectées par le patient pendant son séjour dans la "cabine".Un siège isolant assure le confort du patients sans perturber les distributions de charges environnantes .Une ouverture circulaire en forme de manchon,disposée à mi-hauteur de la cabine et face au patient,reçoit l'extrémité émissive du générateur d'aéro-ions,disposé sur une console extérieure ad hoc .

Un embout disposé sous le manchon reçoit un tube souple relié à une bouteille d'Oxygène,et permet ainsi si nécessaire une suroxygénation du patient sous forme d'oxygène ionisé. Une série d'ouvertures,situées derrière le patient,permet la circulation et l'évacuation de l'air injecté par le générateur .Un capteur électronique permet enfin à tout instant de vérifier la concentration ionique dans l'atmosphère de la cabine .

Le dispositif suivant l'invention permet donc d'assurer l'injection d'un flux déterminé d'aéro-ions oxygène dans une enceinte close,le maintien d'une concentration constante élevée au voisinage du patient,l'absence complète de capture des ions -donc de perte- au niveau de la paroi interne qui se charge négativement et repousse ainsi les aéro-ions vers le patient,l'existence d'un flux ionique constant traversant le patient -nécessairement dévêtu-flux capté simultanément par les voies respiraroires et la surface cutanée et apte à s'écouler normalement par l'électrode plantaire,assurant ainsi le passage indispensable des charges négatives tout au long d'un circuit électrique complet,et autorisant de ce fait l'existence des effets thérapeutiques recherchés .

Il en résulte une amélioration fondamentale et décisive de tous les dispositifs ayant vocation de "cabine d'ionisation" dans toute la mesure où les paramètres physiques fondamentaux sont dorénavant respectés.

Le dispositif qui fait l'objet de l'invention sera décrit plus en détail en se référant aux dessins annexés dans lequels :

- la figure 1 est un schéma (synoptique du dispositif sous la forme des fonctions propres à cahcune de ses parties ;

- la figure 2 est un schéma de principe des distributions électriques à l'intérieur de la cabine ;

- la figure 3 est une vue en perspective d'une réalisation possible sous forme d'une cabine autonome parallélépipédique.

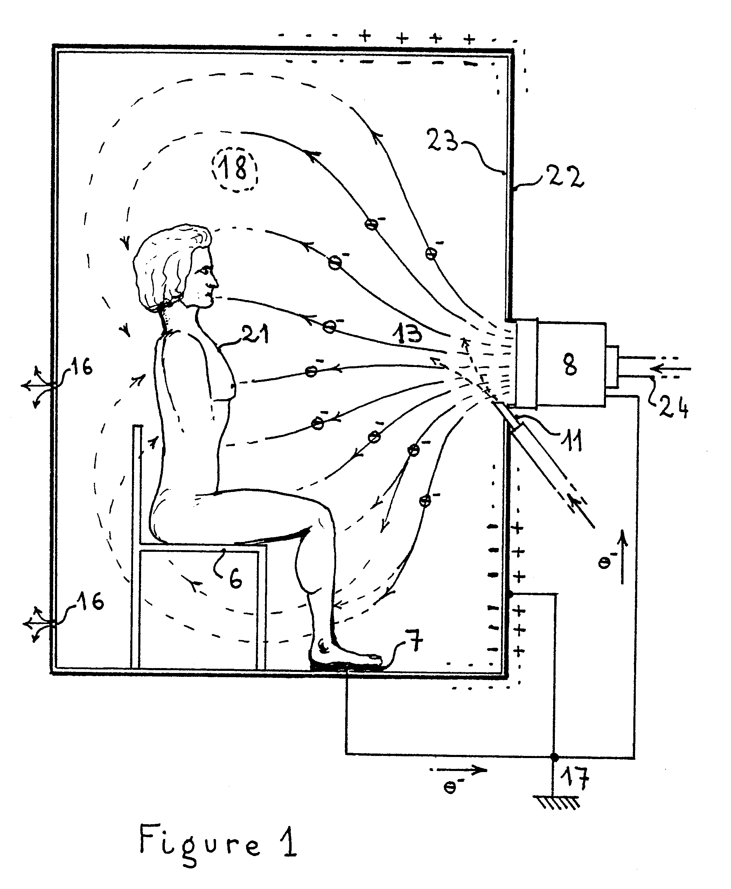

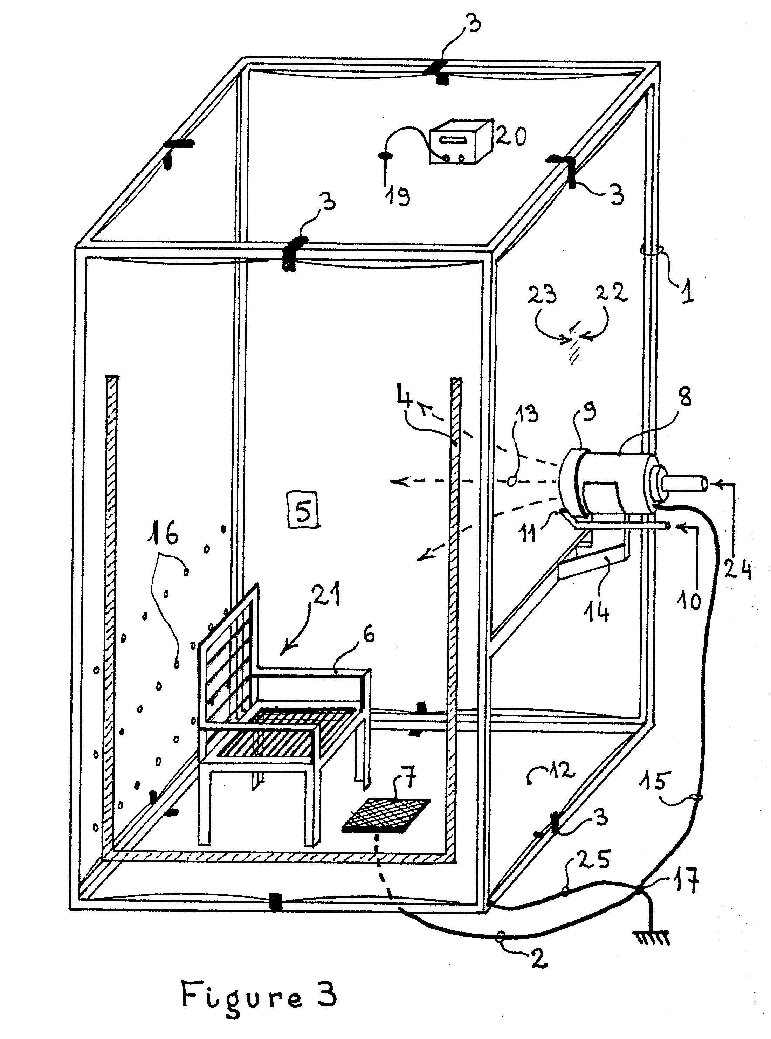

Tel qu'il représenté sur les figures 1 et 3, le dispositif comporte ;

- un générateur d'aéro-ions négatifs (8) du modèle décrit dans la demande de Brevet français No 86 12448 :ce générateur injecte dans la cabine par le manchon (9) les aéro-ions produits (13)

- une "cabine" constituée par une armature métallique extérieure (1) connectée à la masse générale (sol) (17) et au point de potentiel zéro du générateur (8) par la liaison (15);l'armature (1) supporte la cabine proprement dite (18),faite d'un tissu plastique non poreux,de haut isolement,dont la face interne (23) est isolante et la face externe (22) a été rendue conductrice par une métallisation superficielle:cette face externe est également reliée à la masse (17) par les agraffes (3) conductrices assurant la fixation de la cabine sur son armature ;

- une "porte" d'accès (5) vers l'intérieur de la cabine,constituée par l'un des pans latéraux de la-dite cabine,dont la fixation amovible est faite d'un ruban de type "Velcro" (4),d'une fermeture à glissière,ou de tout système assurant une obturation étanche ;

- un siège (6) en matériau isolant très largement ajouré,destiné à recevoir le patient ;
- une plaque (7) de caoutchouc graphité conducteur,ou toute autre substance conductrice équivalente,de surface suffisante pour recevoir la surface plantaire des pieds nus du patient;cette plaque (7) est également reliée à la masse (17) ;
- une bouteille d'Oxygène (10) permettant d'injecter par un orifice (11) une quantité jugée nécessaire d'oxygène dans la cabine; l'orifice (11) permet de plus d'injecter cet oxygène additionnel très près de la source d'électrons (8) et d'assurer de ce fait l'ionisation maximum du-dit oxygène ;
- une sonde conductrice (19) reliée à un appareil électronique de mesure de la densité de charge ionique (20)de la cabine ;
- un dispositif accessoire (24) de réchauffage de l'air injecté,pour le confort du patient et la suppression de la buée .

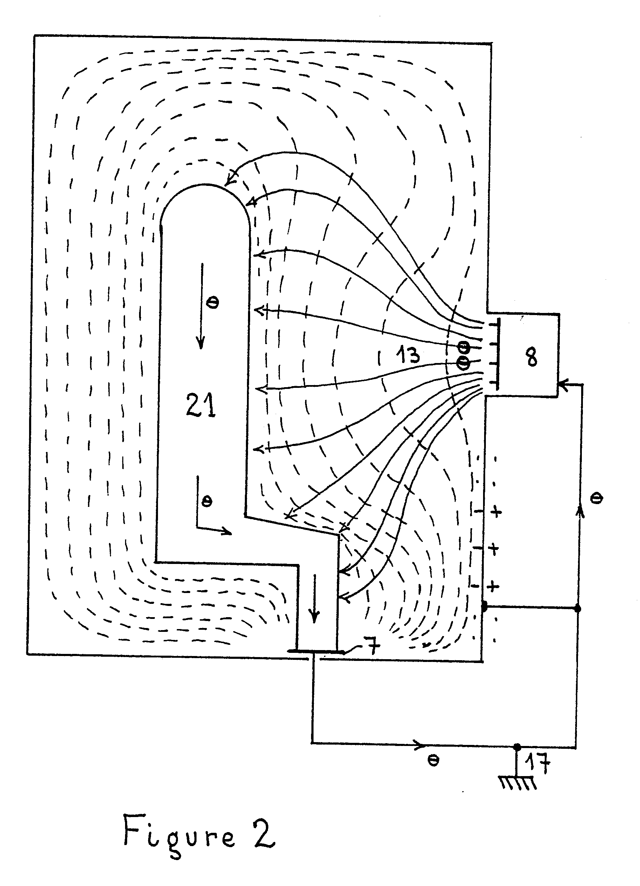

La figure 2 montre la configuration des équipotentielles et des lignes de courant en présence d'un patient (21) (vue en coupe sagittale au centre) .

Le maintien au potentiel zéro de la face externe conductrice (22) de la cabine entretient sur la face interne (23) une densité superficielle négative de valeur élevée(liée à la densité volumique intérieure);cette densité superficielle intérieure assure la répulsion des charges voisines et leur entrainement vers le corps du patient (21) déshabillé,porté lui-même au potentiel zéro par sa liaison avec la plaque conductrice (7) reliée elle-même à la masse (17).

Il en résulte un double flux ionique,correspondant à la circulation permanente d'un double courant électrique :

- flux entraîné par les mouvements respiratoires,avec capture ionique tout au long des cavités bucco-pharyngée,bronchique et alvéolaire ,
- flux permanent de charges entrant par voie cutanée sous l'influence du champ électrique élevé règnant entre les parois chargées négativement et le patient .

Il en résulte obligatoirement la capture -par les voies interne et externe- de la majeure partie des charges produites par le générateur (8),la circulation permanente d'un courant électrique mesurable à l'intérieur du circuit fermé (générateur-atmosphère de la cabine-patient-plaque de caoutchouc plantaire-retour de masse du générateur).

Ce sont les effets conjugués du champ électrique,de l'oxygène ionisé inhalé,des charges captées par l'ensemble de la surface corporelle et des faibles courants qui en résultent,qui permettent d'obtenir et observer les effets hygiéniques ou thérapeutiques attendus .

Un exemple d'application concerne les patients atteints d'affections bronchiques ou trachéo-bronchiques de type asthmatique :une série de 12 à 15 séjours,quotidiens,d'une durée de 20 à 30 minutes est applicable à ces patients.

Un autre exemple d'application concerne les patients atteints d'affections cardio-vasculaire,dont la convalescence est améliorée et accélérée par ce type de traitement .

Ces exemples d'application n'épuisent nullement l'invention qui peut être utilisée dans tous les cas où l'état d'un patient justifie ou requiert des séjours en atmosphère ionisée négativement ,suroxygénée ou non,de façon intensive et répétée .

La figure 3 montre un exemple de réalisation d'une cabine de forme parallèlépipédique autonome,transportable,d'un volume voisin de 4 m³ .

- Une armature métallique légère (profilé en alliage léger) (1) définit un volume de dimensions (1,4/1,4/2 mètres); cette armature est reliée à la masse générale (potentiel zéro), au sol (17),et au point zéro de potentiel du générateur (8) par les liaisons (15) et (25) ;
- sur cette armature est fixée une cabine entièrement étanche (18),faite d'une feuille plastique isolante transparente -ou au moins translucide- dont la face externe des parois (22) a été métallisée(par projection sous vide par exemple) de manière à devenir conductrice,tandis que la face interne (23) des parois demeure strictement isolante;un panneau mobile (5) permet d'accéder à l'intérieur,la fermeture également étanche étant obtenue par des bandes adhésives (4) de type "Velcro" ou tout autre système équivalent .La fixation de la cabine sur l'armature est assurée par agraffes (3) conductrices assurant donc la mise à la masse (17) nécessaire de la paroi externe par le câble (25) ;
- sur la face inférieure (12) de la cabine (le "sol") -faite du même type de tissu que l'ensemble précédent- est fixée une plaque (7) de caoutchouc graphité conducteur,dont la surface de l'ordre de 6 dm² reçoit la plante des pieds nus du patient,en appui franc nécessaire à assurer un contact électrique satisfaisant.La plaque (7) est reliée à la masse (17) par un conducteur (2) ;
- un siège (6) en matériau isolant,très largement ajouré,reçoit le patient (21) nécessairement dévêtu afin d'assurer la capture maximum d'ions par la surface corporelle.La plante des pieds du patient assure alors un contact franc sur la plaque (7) et le retour du courant au point zéro de potentiel du générateur (8) via la masse générale (17) ;
- un générateur d'ions négatifs (8),du modèle décrit dans la demande de Brevet français N° 86 12448 ,injecte le flux d'électrons et d'oxygène ionisé (13) dans la cabine (18) par l'entremise d'un manchon de liaison (9);une console (14) supporte le générateur et ses accessoires éventuels ;
- une arrivée d'air réchauffé (24) dans le générateur (8) permet d'assurer le confort du patient tandis qu'elle évite totalement la formation de buée sur les parois internes (23) de la cabine qui doivent impérativement demeurer sèches et isolantes ;
- une arrivée d'oxygène (11), provenant de la bouteille (10), permet -si le praticien le juge nécessaire- de maintenir l'atmosphère de la cabine suroxygénée,et d'assurer l'ionisation de cet oxygène additionnel par son injection au voisinage immédiat du flux d'électrons (13) issu du générateur (8) ;
- une série d'ouvertures (16) situées dans la paroi arrière de la cabine, destinées à l'évacuation de l'air injecté par le générateur (8),et permettant son renouvellement ;
- un capteur (19) connecté à un module électronique de mesure (20) permet de connaitre en permanence l'ordre de grandeur de la densité d'ions négatifs dans l'atmosphère de la cabine .

Dans les conditions décrites,cette densité d'ions atmosphérique à l'intérieur de la cabine pourra varier entre 0,1 et 10 millions d'ions négatifs par $cm^3$ selon la réglage du générateur (8),le courant ionique total collecté par le patient (21) variant alors de 0,2 à 20 microampères .

Il est évident qu'un tel exemple de réalisation n'épuise nullement l'invention qui peut être réalisée sous forme cylindrique,hémisphérique ...etc...,sous forme d'une cabine fixe à parois solides,transparentes ou opaques,sous forme d'une cabine allongée dans laquelle le patient est couché,ou dans toute autre configuration ou disposition convenable respectant les règles ou exigences résultant de la présence de charges électriques libres dans un milieu gazeux confiné ,telles qu'elles sont rappelées et mises en oeuvre dans le dispositif objet de l'invention.

**Revendications**

1 - Dispositif pour aéro-ionisation négative en atmosphère contrôlée compotant un générateur (8) d'ions oxygène négatifs injecte les-dits ions dans une cabine (18) close à parois isolantes,un patient dévêtu,placé à l'intérieur de la-dite cabine,relié à la masse générale (17) du dispositif, collectant par voie respiratoire et capture corporelle les ions négatifs issus du générateur (8) et une source auxiliaire (11)permettant si nécessaire d'accroître le taux d'oxygène ionisé présent dans la cabine, dispositif comportant deux sous- ensembles autonomes :
- un générateur d'aéro-ions négatifs (8) et ses accessoires (source d'Oxygène,source d'air réchauffé), nécessaires au second sous-ensemble ;
- une cabine en matériau isolant (18) dont les caractéristiques assurent au patient (21) la capture maximum et continue des aéro-ions émis par le générateur (8),et de ce fait la possibilité de bénéficier des propriétés hygiéniques ou thérapeu tiques des-dits aéro-ions sans aucune restriction .

2 - Dispositif suivant la revendication 1, comportant un générateur (8) d'aéro-ions de grande puissance,à débit variable,utilisable en milieu confiné (absence d'ozone et d'oxydes d'azote),pourvu d'une alimentation extérieure d'air réchauffé ,et d'une source accessoire d'Oxygène .

3 - Dispositif suivant la revendication 1, comportant une cabine en matériau isolant,dont la face externe (22) rendue conductrice est reliée à la masse de potentiel zéro (17) commune au générateur (8) et au sol,et dont la face interne isolante (23) acquiert une charge superficielle négative créant entr'elle et le patient (21) un champ électrique imposant aux ions atmosphériques présents un mouvement aboutissant à leur capture par le-dit patient (21) et leur retour vers la-dite masse commune (17) et le-dit générateur (8).

4 - Dispositif suivant la revendication 1, conformément au quel le patient (21) est placé au centre de la cabine (18) sur un support isolant,le-dit patient étant nécessairement porté au potentiel zéro (masse 17) par le contact (7) d'une plaque conductrice connectée à la-dite masse,de manière à assurer la collecte et la capture quasi-complète par le patient (21) des charges produites par le générateur (8) .

5 - Dispositif suivant la revendication 4, comportant une plaque (7) conductrice,placée sous l'espace plantaire du patient (21),destinée à recevoir les charges collectées par le-dit patient et les acheminer vers la masse commune (17) et le retour au générateur (8) .

6 - Dispositif suivant la revendication 5, dans lequel la configuration de champ électrique à l'intérieur de la cabine (18) assure une double capture des ions négatifs par le patient (21) : par les voies respiratoires d'une part,par la surface corporelle d'autre part,la perte d'ions résiduelle restant de ce fait très faible .

7 - Dispositif suivant la revendication 6, dans lequel l'absence de pertes résiduelles élevées permet un dosage convenable des quantités d'ions attribuées aux différents patients en fonction de leur état ou de leur besoin propre,et le contrôle permanent des-dites quantités par l'entremise du capteur-module (19-20) .

8 - Dispositif suivant la revendication 1, comportant une injection séparée d'Oxygène (11) préservant le générateur (8) de tout risque d'oxydation,mais assurant l'ionisation maximum du-dit Oxygène par son injection au voisinage extérieur des pointes émissives du générateur (8) .

9 - Dispositif suivant la revendication 8, comportant des ouvertures (16) nombreuses et de faible diamètre,destinées à assurer l'évacuation de l'air injecté en (9) et (11) sans entraîner de turbulences dans l'atmosphère de la cabine et sans perturber de ce fait la distribution et la capture des charges présentes .

10 - Dispositif suivant la revendication 9, dont la structure et le fonctionnement autorisent l'utilisation dans les domaines biologique ou biomédical à des fins hygiéniques ou thérapeutiques lors du séjour , dans une atmosphère confinée chargée d'ions négatifs de densité volumique prédéterminée,d'un patient susceptible de capter,transporter et écouler les charges électriques résultant du-dit séjour dans la-dite atmosphère ionisée .

0267077

Figure 1

0267077

Figure 2

Figure 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | WO-A-8 000 918 (PRIORE) <br> * Page 8, ligne 9 - page 9, ligne 37; figure 1 * | 1,4-6, 10 | A 61 N 1/44 |
| A | | 2,3,7-9 | |
| | --- | | |
| A | FR-A-2 258 598 (FURCHNER) <br> * Page 5, ligne 6 - page 6, ligne 17 * | 1-10 | |
| | --- | | |
| A | FR-A-2 262 423 (OZONAIR) <br> * Page 3, ligne 13 - page 4, ligne 21 * | 1,2 | |
| | ----- | | |

**DOMAINES TECHNIQUES RECHERCHES (Int. Cl.4)**

A 61 N

**Le présent rapport a été établi pour toutes les revendications**

| Lieu de la recherche | Date d'achèvement de la recherche | Examinateur |
|---|---|---|
| LA HAYE | 07-01-1988 | LEMERCIER D.L.L. |

**CATEGORIE DES DOCUMENTS CITES**

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
................................................................
& : membre de la même famille, document correspondant

EPO FORM 1503 03.82 (P0402)